(19) Europäisches Patentamt
European Patent Office
Office européen des brevets

(11) Publication number: 0 499 130 A1

# (12) EUROPEAN PATENT APPLICATION

(21) Application number: **92101913.9**

(22) Date of filing: **05.02.92**

(51) Int. Cl.[5]: **C07D 487/04**, A61K 31/40, //(C07D487/04,209:00,209:00)

(30) Priority: **15.02.91 JP 21243/91**

(43) Date of publication of application: **19.08.92 Bulletin 92/34**

(84) Designated Contracting States: **DE FR GB IT**

(71) Applicant: **KYOWA HAKKO KOGYO CO., LTD.**
**6-1, Ohte-Machi Itchome**
**Chiyoda-ku Tokyo(JP)**

(72) Inventor: **Saito, Hiromitsu**
**2-20-10, Higashifuchinobe**
**Sagamihara-shi, Kanagawa-ken(JP)**
Inventor: **Nagamura, Satoru**
**3-9-9, Naka-machi**
**Machida-shi, Tokyo(JP)**
Inventor: **Kobayashi, Eiji**
**8-6-106, Takasago-cho**
**Numazu-chi, Shizuoka-ken(JP)**
Inventor: **Gomi, Katsushige**
**541-12, Izu-shimada**
**Susono-shi, Shizuoka-ken(JP)**

(74) Representative: **Casalonga, Axel et al**
**BUREAU D.A. CASALONGA - JOSSE**
**Morassistrasse 8**
**W-8000 München 5(DE)**

## (54) DC-89 derivatives.

(57) DC-89 derivatives represented by the formula:

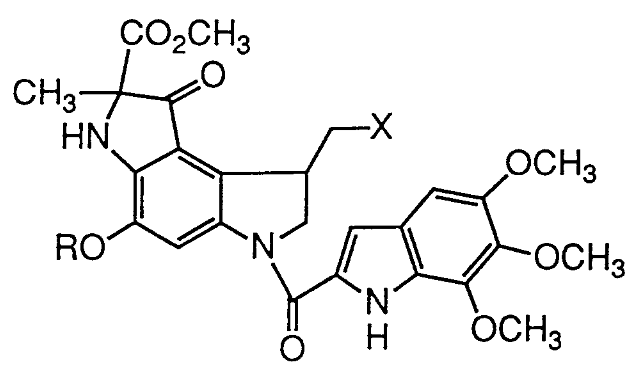

wherein X represents chlorine or bromine; and R represents

$-SO_2R^1$ in which $R^1$ represents a straight-chain or branched alkyl group having 1 to 6 carbon atoms or phenyl group, or

$-CO(CH2)_nR^2$ in which n represents an integer of 0 to 5, and $R^2$ represents

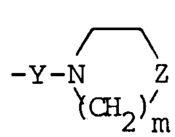

EP 0 499 130 A1

in which m represents an integer of 1 to 4;
Y represents a single bond or CO; and Z represents $CH_2$, O or N-$R^3$ (in which $R^3$ represents hydrogen or a straight-chain or branched alkyl group having 1 to 6 carbon atoms) or

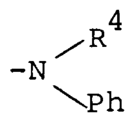

in which $R^4$ represents hydrogen or a straight-chain or branched alkyl group having 1 to 6 carbon atoms, and Ph represents phenyl group

and pharmaceutically acceptable salts thereof have an excellent anti-tumor activity and are expected to be useful as anti-tumor compositions.

Background of the Invention

The present invention relates to DC-89 derivatives. The compounds have an excellent anti-tumor activity and are expected to be useful as anti-tumor agents.

As compounds similar to the DC-89 derivatives of the present invention, DC-89A1, DC-89A2, DC-89B1 and DC-89B2 represented by the following structural formula are known. These compounds exhibit an antibacterial activity against various bacteria and also show an anti-tumor activity against melanoma B-16, etc.

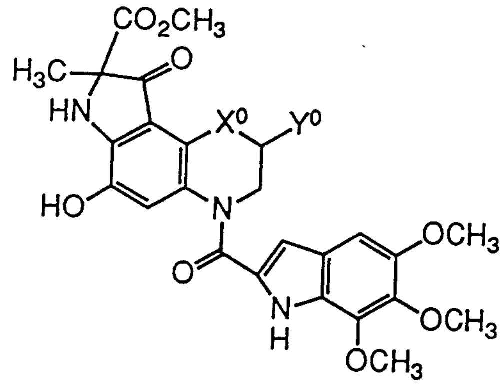

DC-89A1: $X^0$ = $-CH_2-$, $Y^0$ = Cl
DC-89A2: $X^0$ = single bond, $Y^0$ = $CH_2Cl$
DC-89B1: $X^0$ = $-CH_2-$, $Y^0$ = Br
DC-89B2: $X^0$ = single bond, $Y^0$ = $CH_2Br$

DC-89A1 is disclosed in WO 87/06265 (EP-A-0271581); and DC-89A2, DC-89B1 and DC-89B2 are disclosed in Japanese Published Unexamined Patent Application No. 119787/90 (EP-A-0351865). SF2582A and SF2582B which are the same compounds as DC-89A2 and DC-89A1 respectively are disclosed in Japanese Published Unexamined Patent Application No. 139590/89 (EP-A-0318056). DC-88A having the following structure which is relevant to the compounds of the present invention is disclosed in WO 87/06265. DC-88A not only shows an anti-bacterial activity against a variety of bacteria but also exhibits an anti-tumor activity against melanoma B-16, etc.

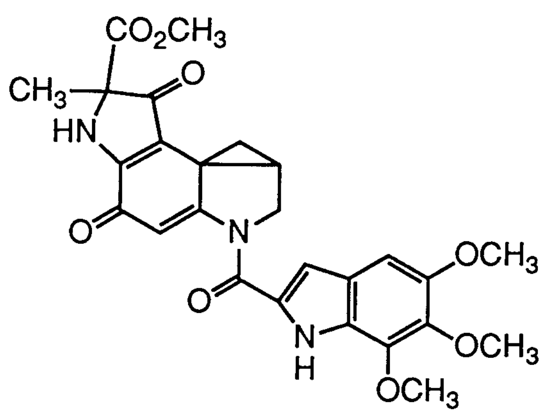

DC-88A derivatives are disclosed in Japanese Published Unexamined Patent Application No. 288879/90 (EP-A-0354583), Japanese Published Unexamined Patent Application No. 7287/91 (EP-A-0365041) and Japanese Published Unexamined Patent Application No. 128379/91 (EP-A-0406749). These derivatives are also disclosed in EP-A-0461603 and EP-A-0468400.

Further, as compounds which are structurally similar to the compounds of the present invention, derivatives of SF2582C are disclosed in Japanese Published Unexamined Patent Application No. 275581/89 (EP-A-0339681) and CC-1065 and its derivatives arc disclosed in Japanese Published Unexamined Patent Application No. 64695/79 (USP 4,169,888), Japanese Published Unexamined Patent Application No.

193989/85 (EP-A-0154445), WO 88/04659 and EP-A-359454.

## Summary of the Invention

The present invention relates to DC-89 derivatives represented by formula (I):

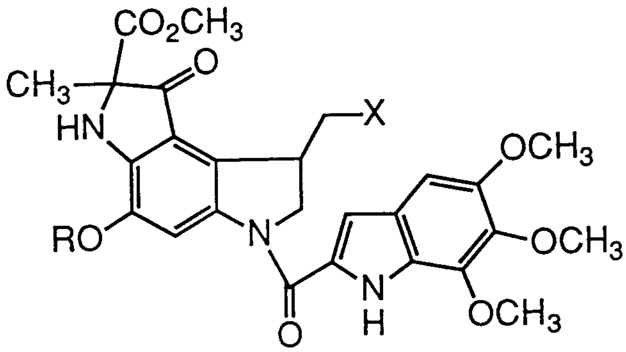

(I)

wherein X represents chlorine or bromine; and R represents

$-SO_2R^1$ in which $R^1$ represents a straight-chain or branched alkyl group having 1 to 6 carbon atoms or phenyl group, or

$-CO(CH_2)_nR^2$ in which n represents an integer of 0 to 5, and $R^2$ represents

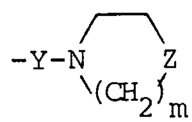

in which m represents an integer of 1 to 4;
Y represents a single bond or CO; and Z represents $CH_2$, O or $N-R^3$ (in which $R^3$ represents hydrogen or a straight-chain or branched alkyl group having 1 to 6 carbon atoms) or

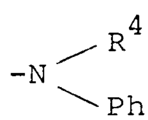

in which $R^4$ represents hydrogen or a straightchain or branched alkyl group having 1 to 6 carbon atoms, and Ph represents phenyl group

and pharmaceutically acceptable salts thereof.

## Detailed Description of the Invention

The compounds represented by general formula (I) are hereinafter referred to as Compounds (I). Similarly, the compounds represented by general formulae (II) through (IV) are referred to as Compounds (II) through (IV). Compounds (I)a, (I)b, etc. are intended to be included in Compounds (I); Compounds (I)b1, (I)b2, etc. are intended to be included in Compounds (I)b; and Compounds (I)bl-1 are intended to be included in Compounds (I)b1. In the definition of R in general formula (I), the straight-chain or branched alkyl group having 1 to 6 carbon atoms include, for example, methyl, ethyl, propyl, isopropyl, butyl, isobutyl, sec-butyl, tert-butyl, pentyl and hexyl.

As the pharmaceutically acceptable salts of Compounds (I), inorganic acid-addition salts such as hydrochloride, hydrobromide, hydroiodide, sulfate, phosphate and nitrate, and organic acid-addition salts such as acetate, benzoate, maleate, fumarate, succinate, tartrate, citrate, oxalate, glyoxylate, aspartate and methanesulfonate may be mentioned.

The processes for preparing Compounds (I) are described below.

Process 1

Compounds (I)a [Compounds (I) wherein R is $-SO_2R^1$] can be prepared according to the following step:

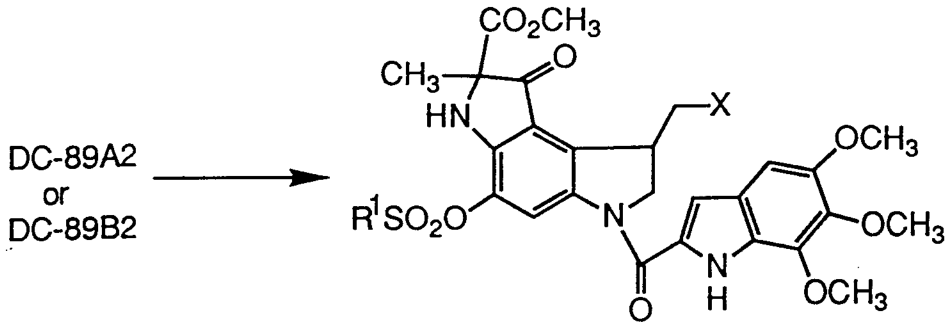


(I)a

In this formula, X and $R^1$ have the same significances as defined above.

Compound (I)a can be prepared by allowing DC-89A2 or DC-89B2 obtained by culturing a microorganism belonging to the genus Streptomyces to react with Compound (II) represented by the following formula:

$R^1SO_2$-Hal (II)

(wherein Hal represents chlorine, bromine or iodine, and $R^1$ has the same significance as defined above) in an inert solvent in the presence of a base. As the base, triethylamine, pyridine, 4-dimethylaminopyridine, etc. may be used. The base is usually used in an amount of 1 to 5 equivalents based on DC-89A2 or DC-89B2, but when the base serves also as a solvent, it is used in large excess of DC-89A2 or DC-89B2. As the inert solvent, pyridine, dichloromethane, dimethylformamide, tetrahydrofuran (THF), toluene, etc. may be used singly or in combination. Compound (II) is usually used in an amount of 1 to 5 equivalents based on DC-89A2 or DC-89B2. The reaction is carried out at -10 °C to 50 °C and is completed in one hour to one day.

Process 2

Compounds (I)b [Compounds (I) wherein R is:

$-CO(CH_2)_nR^2$

wherein $R^2$ and n have the same significances as defined above] can be prepared by the following process.

Process 2-1

Compounds (I)b1 [Compounds (I)b wherein n is 0] can be prepared according to the following steps.

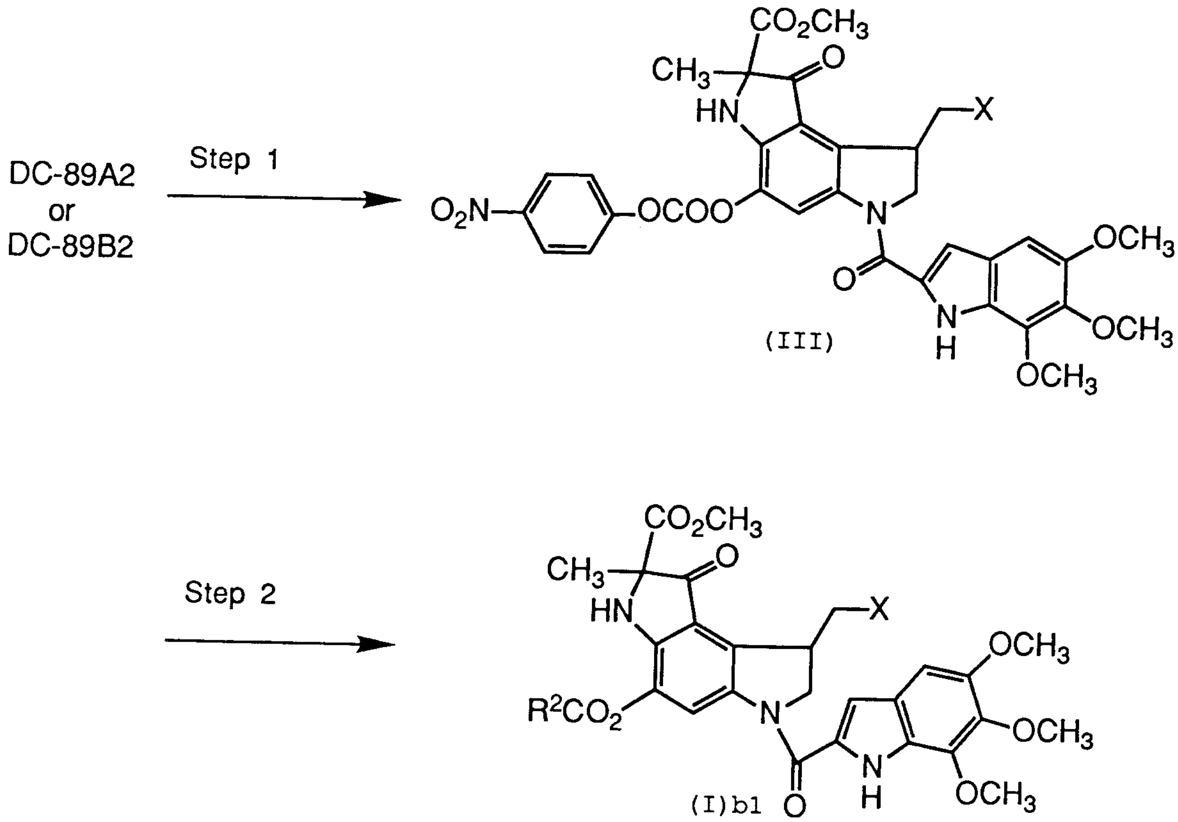


(III)

(I)b1

In these formulae, X and $R^2$ have the same significances as defined above.

(Step 1)

Compound (III) can be obtained by allowing DC-89A2 or DC-89B2 to react with p-nitrophenyl chloroformate in an inert solvent in the presence of a base. As the base, triethylamine, pyridine, 4-dimethylaminopyridine, etc. may be used. The base is usually used in an amount of 1 to 5 equivalents based on DC-89A2 or DC-89B2, but when the base serves also as a solvent, it is used in large excess of DC-89A2 or DC-89B2. As the inert solvent, pyridine, dichloromethane, dimethylformamide, THF, toluene, etc. may be used singly or in combination. p-Nitrophenyl chloroformate is usually used in an amount of 1 to 5 equivalents based on DC-89A2 or DC-89B2. The reaction is carried out at -10 °C to 50 °C and is completed in 30 minutes to 5 hours.

(Step 2)

Compound (III) is then allowed to react with Compound (IV) represented by the following formula:

$R^2$-H (IV)

(wherein $R^2$ has the same significance as defined above) in an inert solvent to obtain Compound (I)b1. As the inert solvent, pyridine, dichloromethane, dimethylformamide, THF, toluene, etc. may be used singly or in combination. Compound (IV) is usually used in an amount of 1 to 5 equivalents based on Compound (III). The reaction is carried out at -10 °C to 50 °C and is completed in 30 minutes to 5 hours.

(Process 2-2)

Compounds (I)b1-1 [Compounds (I)b1 wherein $R^2$ is PhNH] can also be prepared according to the following step:

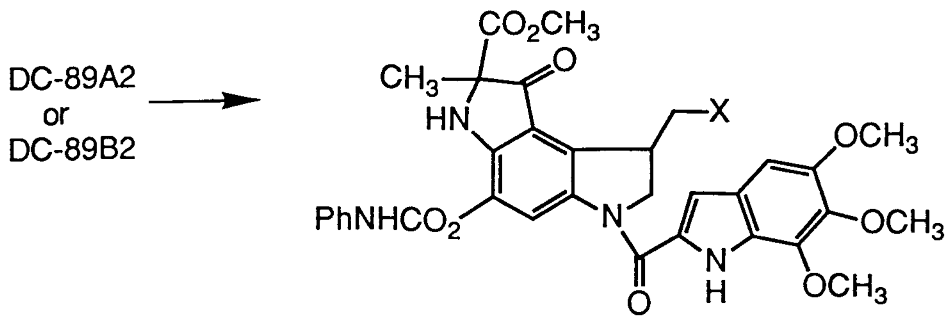


(I)b1-1

In this formula, X and Ph have the same significances as defined above.

Compound (I)b1-1 can be prepared by allowing DC-89A2 or DC-89B2 to react with phenyl isocyanate in an inert solvent in the presence of a base. As the base, triethylamine, pyridine, 4-dimethylaminopyridine, etc. may be used. The base is usually used in an amount of 1 to 3 equivalents based on DC-89A2 or DC-89B2. Phenyl isocyanate is usually used in an amount of 1 to 3 equivalents based on DC-89A2 or DC-89B2. As the inert solvent, dichloromethane, dimethylformamide, THF, toluene, etc. may be used singly or in combination. The reaction is carried out at -20 ° C to 50 ° C and is completed in 30 minutes to 3 hours.

Process 2-3

Compounds (I)b2 [Compounds (I)b wherein n is 1 to 5] can be prepared according to the following step:

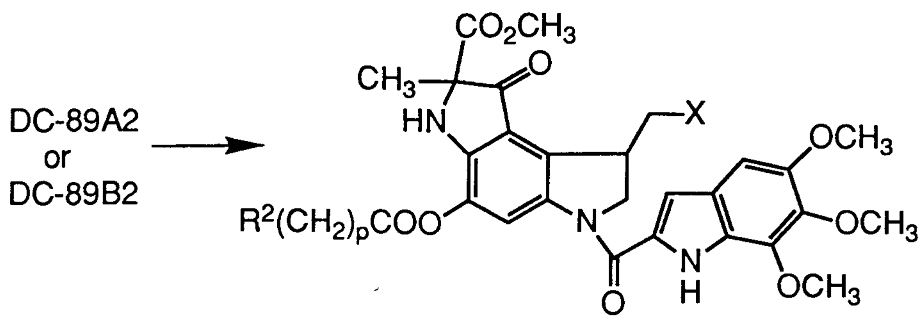


(I)b2

In this formula, p represents an integer of 1 to 5, and X and $R^2$ have the same significances as defined above.

Compound (I)b2 can be prepared by allowing DC-89A2 or DC-89B2 to react with Compound (V) represented by the following formula:

$R^2(CH_2)_pCOOH$ (V)

(wherein $R^2$ and p have the same significances as defined above) in an inert solvent in the presence of dicyclohexylcarbodiimide (DCC). Compound (V) and DCC are usually used in an amount of 1 to 5 equivalents each based on DC-89A2 or DC-89B2. The reaction is carried out at -10 ° C to 50 ° C and is completed in 30 minutes to one day.

After completion of the reaction in each step, water, an acid or a buffer solution may be added to the reaction mixture, if necessary, followed by extraction with a water-immiscible solvent such as ethyl acetate, chloroform or ether. The extract is washed with water, an aqueous solution of sodium chloride, or the like, and dried over anhydrous sodium sulfate, or the like. Then, the solvent is distilled off, and the residue is subjected to silica gel column chromatography, thin layer chromatography, high performance liquid

preparative chromatography, recrystallization, or the like to effect purification.

In the case where a salt of Compound (I) is desired and it is produced in the form of the desired salt, it can be subjected to purification as such. In the case where Compound (I) is produced in the free state and its salt is desired, Compound (I) can be converted into its salt by dissolving or suspending the compound in an appropriate solvent and adding a suitable acid to the solution or suspension.

Intermediates may be directly used in the subsequent reaction without being isolated or purified. Compounds (I) and pharmaceutically acceptable salts thereof may be in the form of adducts with water or various solvents, which are also within the scope of the present invention. Furthermore, all possible stereoisomers of Compounds (I) including optical isomers and mixtures thereof also fall within the scope of the present invention.

The structures and compound numbers of representative compounds which fall under Compounds (I) are shown in Table 1.

Table 1

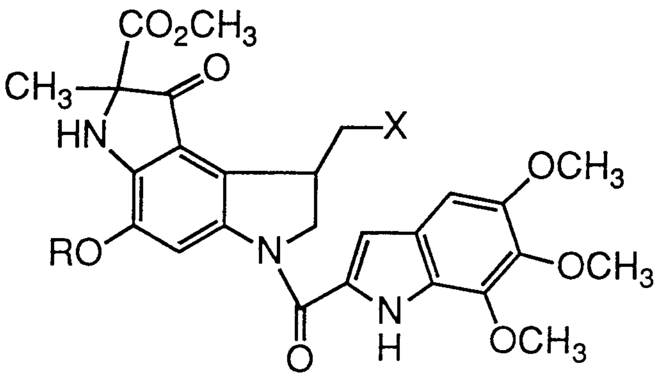

| Compound No. | R | X |
|---|---|---|
| 1 | $CH_3SO_2$ | Br |
| 2 | 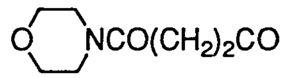 | Br |
| 3 | 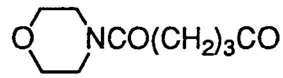 | Br |
| 4 | 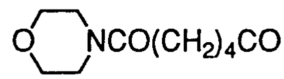 | Br |
| 5 | 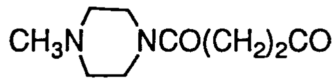 | Br |
| 6 | 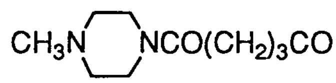 | Br |
| 7 | 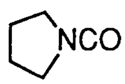 | Br |
| 8 | 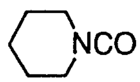 | Br |
| 9 | 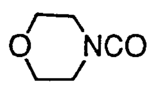 | Br |
| 10 | 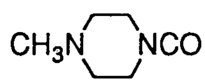 | Br |
| 11 | 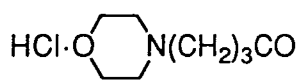 | Br |
| 12 | 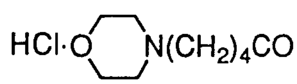 | Br |
| 13 | 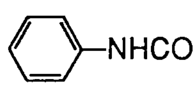 | Br |
| 14 | 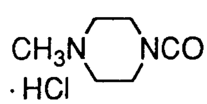 | Br |
| 15 | 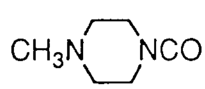 | Cl |

The pharmacological activity of representative Compounds (I) is shown below.

Growth Inhibitory Effect against $HeLaS_3$ Cells

$HeLaS_3$ cells were suspended in a medium comprising MEM medium, 10% fetal calf serum and 2 mM

glutamine (hereinafter referred to as medium A) to a concentration of 3 x$10^4$ cells/ml. The cell suspension thus prepared was put into wells of a 96-well microtiter plate in the amount of 0.1 ml per well. After incubation at 37° C for 20 hours in a $CO_2$-incubator, 0.05 ml of a test sample containing Compound (I) appropriately diluted with medium A was added to each well.

The cells were further cultured at 37° C for 72 hours in the $CO_2$-incubator and the culture supernatant was removed. After the residue was washed once with phosphate buffer saline (PBS), a medium comprising medium A and 0.02% Neutral Red was added in the amount of 0.1 ml per well. Then, the cells were cultured at 37° C for one hour in the $CO_2$-incubator, whereby the cells were stained. After removal of the culture supernatant, the residue was washed once with physiological saline. The pigment was extracted with 0.001N hydrochloric acid/30% ethanol and the absorbance of the extract at 550 nm was measured by using a microplate reader. The concentration of the test compound at which the growth of the cells is inhibited by 50% ($IC_{50}$) was calculated by comparing the absorbance of untreated cells with those of the cells treated with the test compound at known concentrations. The result is shown in Table 2.

Therapeutic Effect against Sarcoma 180 Tumor

Five male ddY-strain mice each weighing 18 to 20 g were used for each group as test animals, and 5 x$10^5$ Sarcoma 180 tumor cells were implanted subcutaneously into the animals at the axilla. One day after the implantation, 0.2 ml of physiological saline containing Compound (I) at the concentration indicated in Table 2 was intravenously administered to each mouse. T/C [T: average tumor volume (mm3) of the group treated with the test compound, C: average tumor volume ($mm^3$) of the control group which received an intravenous administration of 0.2 ml of physiological saline] was determined seven days after the implantation.

The result is shown in Table 2.

Table 2

| Compound No. | $IC_{50}$ (nM) | Dose (mg/kg) | T/C |
|---|---|---|---|
| 1 | 5.8 | | |
| 2 | 0.054 | | |
| 3 | 0.18 | | |
| 4 | 0.092 | | |
| 5 | 2.8 | | |
| 6 | 0.25 | | |
| 7 | 0.23 | | |
| 8 | 6.6 | 0.50 | 0.13 |
| 9 | 30 | 0.50 | 0.13 |
| 10 | 71 | 1.0 | 0.12 |
| 14 | 15 | 1.0 | 0.13 |

Acute Toxicity Test

A test compound was intraperitoneally administered to ddY-strain male mice each weighing 20 ± 1 g. MLD (the minimum lethal dose) was determined by observing the mortality for 14 days after the administration.

The result is shown in Table 3.

Table 3

| Compound No. | Acute Toxicity (MLD) mg/kg |
|---|---|
| 1 | >8.0 |
| 2 | 0.50 |
| 3 | 0.50 |
| 4 | 1.0 |
| 5 | >8.0 |
| 6 | 8.0 |
| 7 | 1.0 |
| 8 | 1.0 |
| 9 | 0.50 |
| 10 | 1.0 |
| 13 | 0.50 |
| 14 | 1.0 |

Compounds (I) and pharmaceutically acceptable salts thereof may be used as anti-tumor agents, singly or in combination with at least one pharmaceutically acceptable carrier. For example, Compounds (I) or salts thereof are dissolved in a physiological saline or in an aqueous solution of glucose, lactose, mannitol, or the like to prepare a pharmaceutical composition suitable for injection. Alternatively, Compounds (I) or salts thereof are freeze-dried in a conventional manner and mixed with sodium chloride to prepare a powder injection. If necessary, the pharmaceutical composition may contain additives well known in the art of medical preparation, for example, pharmaceutically acceptable salts.

Although the dose of the composition may vary depending upon the age, condition, etc. of the patient, it is suitable to administer Compound (I) in a dose of 0.01 to 20 mg/kg/day for mammals including human beings. Administration may be made, for example, once a day (single administration or consecutive administrations) or intermittently 1 to 3 times a week or once every 2 to 3 weeks, intravenously. If desired, intraarterial administration, intraperitoneal administration, intrathoracical administration, etc. are also possible in a similar dose and in a similar manner. Further, if desired, the composition may also be administered orally, in a similar dose and in a similar manner. Forms for oral administration include tablets, capsules, powders, granules and ampoules, which contain pharmaceutical auxiliaries well known in the art of medical preparation.

Certain specific embodiments of the present invention are illustrated by the following examples and reference examples.

The physicochemical properties of the compounds shown in the following examples and reference examples were determined with the following equipments.

NMR Bruker AM-400 (400 MHz)
MS Hitachi Ltd. M-80B
IR Japan Spectral Co., Ltd. IR-810

As the silica gel, Wakogel C-200® manufactured by Wako Pure Chemical Industries Co., Ltd. was used.

Example 1 Synthesis of Compound 1

In 10 ml of pyridine was dissolved 50 mg (0.085 mmol) of DC-89B2, and 0.013 ml (0.17 mmol) of methanesulfonyl chloride was added to the solution at 0°C. The mixture was stirred at 0°C to room temperature for 4 hours. Then, 1 N hydrochloric acid was added to the reaction mixture, followed by extraction with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (20 ml of silica gel, n-hexane :ethyl acetate = 4 : 1) to give 42 mg (yield 74%) of Compound 1.

The physicochemical properties of Compound 1 are as follows.

$^1$H-NMR (400MHz, $CDCl_3$) δ (ppm); 9.35(1H, br s), 8.59 (1H, s), 6.96(1H, d, J=2.3Hz), 6.87(1H, s), 5.73 (1H, br s), 4.64(1H, dd, J=10.9, 4.6Hz), 4.59(1H, br d, J=9.4Hz), 4.26(1H, m), 4.08(3H, s), 4.01 (1H, dd, J=10.2, 3.4Hz), 3.94(3H, s), 3.91(3H, s), 3.77(3H, s), 3.68(1H, dd, J=10.1, 10.1Hz), 3.32 (3H, s), 1.70(3H, s)

| | |
|---|---|
| IR (KBr) ν ($cm^{-1}$); | 2934, 1745, 1700, 1618, 1524, 1489, 1367, 1308, 1176, 1117, 1036 |
| SIMS (m/z); | 668, 666$(M+1)^+$, 590, 588, 435, 433, 234 |

Example 2 Synthesis of Compound 2

In 10 ml of dichloromethane was dissolved 150 mg (0.25 mmol) of DC-89B2, and 5 ml of a solution of 78.9 mg (0.38 mmol) of DCC in dichloromethane was added to the solution at 0°C. The mixture was stirred at 0°C for 0.5 hour. Then, 5 ml of a solution of 500 mg (2.67 mmol) of 3-(4-morpholinocarbonyl)propionic acid in dichloromethane was added to the reaction mixture, and the mixture was stirred at 0°C to room temperature for 18 hours. After the reaction mixture was filtered, water was added to the filtrate, followed by extraction with dichloromethane. The dichloromethane layer was washed with 1 N hydrochloric acid, a saturated aqueous solution of sodium hydrogencarbonate and a saturated aqueous solution of sodium chloride, dried over anhydrous sodium sulfate, and then concentrated under reduced pressure. The resulting crude product was dissolved in a small quantity of ethyl acetate and the solution was stirred at 0°C for one hour. After the reaction mixture was filtered, the filtrate was concentrated under reduced pressure. The resulting crude product was purified by silica gel column chromatography (50 ml of silica gel, chloroform methanol = 50 : 1) to give 80.6 mg (yield 42%) of Compound 2.

The physicochemical properties of Compound 2 are as follows.

| | |
|---|---|
| $^1$H-NMR (400MHz, $CDCl_3$) δ (ppm); | 9.32(1H, br s), 8.39 (1H, s), 6.94(1H, d, J=2.3Hz), 6.87(1H, s), 6.70 (1H, br s), 4.62(1H, br d, J=9.4Hz), 4.56(1H, dd, J=10.8, 4.5Hz), 4.22(1H, m), 4.08(3H, s), 3.94 (3H, s), 3.91(3H, s), 3.71-(3H, s), 3.69(5H, m), 3.61(3H, m), 3.51(2H, m), 2.86(4H, m), 1.63-(3H, s) |
| IR (KBr) ν ($cm^{-1}$); | 2934, 1745, 1696, 1627, 1495, 1435, 1307, 1219, 1115, 1033 |
| SIMS (m/z); | 759, 757$(M+1)^+$, 590, 588, 234 |

Example 3 Synthesis of Compound 3

In 10 ml of dichloromethane was dissolved 150 mg (0.25 mmol) of DC-89B2, and 5 ml of a solution of 78.9 mg (0.38 mmol) of DCC in dichloromethane was added to the solution at 0°C. The mixture was stirred at 0°C for 0.5 hour. Then, 77 mg (0.38 mmol) of 4-(4-morpholinocarbonyl)-butyric acid was added to the reaction mixture, and the mixture was stirred at 0°C to room temperature for 18 hours. After the reaction mixture was filtered, water was added to the filtrate, followed by extraction with dichloromethane. The dichloromethane layer was washed with 1 N hydrochloric acid, a saturated aqueous solution of sodium hydrogen-carbonate and a saturated aqueous solution of sodium chloride, dried over anhydrous sodium sulfate, and then concentrated under reduced pressure. The resulting crude product was dissolved in a small quantity of ethyl acetate and the solution was stirred at 0°C for one hour. After the reaction mixture was filtered, the filtrate was concentrated under reduced pressure. The resulting crude product was purified by silica gel column chromatography (50 ml of silica gel, chloroform : methanol = 10 : 1) to give 97.4 mg (field 50%) of Compound 3.

The physicochemical properties of Compound 3 are as follows.

| | |
|---|---|
| $^1$H-NMR (400MHz, $CDCl_3$) δ (ppm); | 9.33(1H, br s), 8.43 (1H, s), 6.95(1H, d, J=2.3Hz), 6.87(1H, s), 6.10 (1H, br s), 4.63(1H, br d, J=9.3Hz), 4.57(1H, dd, J=10.8, 4.6Hz), 4.26(1H, m), 4.08(3H, s), 3.94 (3H, s), 3.91(3H, s), 3.75-(3H, s), 3.67(5H, m), 3.62(3H, m), 3.49(2H, m), 2.75(2H, t, J=6.4Hz), 2.52(2H, t, J=6.4Hz), 2.13(2H, m), 1.67(3H, s) |
| IR (KBr) ν ($cm^{-1}$); | 2938, 1746, 1700, 1618, 1558, 1495, 1429, 1388, 1308, 1114, 1034 |
| SIMS (m/z); | 773, 771$(M+1)^+$, 590, 588, 234 |

Example 4 Synthesis of Compound 4

In 5 ml of dichloromethane was dissolved 50 mg (0.085 mmol) of DC-89B2, and 2 ml of a solution of 27 mg (0.13 mmol) of DCC in dichloromethane was added to the solution at 0°C. The mixture was stirred at 0°C for 0.5 hour. Then, 2 ml of a solution of 50 mg (0.23 mmol) of 5-(4-morpholinocarbonyl)valeric acid in dichloromethane was added to the reaction mixture, and the mixture was stirred at 0°C to room temperature for 18 hours. After the reaction mixture was filtered, water was added to the filtrate, followed by

extraction with dichloromethane. The dichloromethane layer was washed with 1 N hydrochloric acid, a saturated aqueous solution of sodium hydrogencarbonate and a saturated aqueous solution of sodium chloride, dried over anhydrous sodium sulfate, and then concentrated under reduced pressure. The resulting crude product was dissolved in a small quantity of ethyl acetate and the solution was stirred at 0°C for one hour. After the reaction mixture was filtered, the filtrate was concentrated under reduced pressure. The resulting crude product was purified by silica gel column chromatography (30 ml of silica gel, chloroform methanol = 100 : 1) to give 24 mg (yield 36%) of Compound 4.

The physicochemical properties of Compound 4 are as follows.

| | |
|---|---|
| $^1$H-NMR (400MHz, $CDCl_3$) δ(ppm); | 9.32(1H, br s), 8.46 (1H, s), 6.95(1H, d, J = 2.3Hz), 6.87(1H, s), 6.18 (1H, br s), 4.63(1H, br d, J = 9.3Hz), 4.57(1H, dd, J = 10.8, 4.6Hz), 4.23(1H, m), 4.08(3H, s), 3.94 (3H, s), 3.91(3H, s), 3.73(3H, s), 3.69(5H, m), 3.62(3H, m), 3.49(2H, m), 2.68(2H, t, J = 6.5Hz), 2.39(2H, t, J = 6.4Hz), 1.84(4H, m), 1.66(3H, s) |
| IR (KBr) ν ($cm^{-1}$); | 2936, 1743, 1617, 1520, 1493, 1387, 1306, 1234, 1114 |
| SIMS (m/z); | 787, 785$(M+1)^+$, 590, 588, 234 |

Example 5 Synthesis of Compound 5

In 10 ml of dichloromethane was dissolved 100 mg (0.17 mmol) of DC-89B2, and 5 ml of a solution of 53 mg (0.26 mmol) of DCC in dichloromethane was added to the solution at 0°C. The mixture was stirred at 0°C for 0.5 hour. Then, 51 mg (0.26 mmol) of 3-(4-methyl-1-piperazinyl-carbonyl)propionic acid was added to the reaction mixture, and the mixture was stirred at 0°C to room temperature for 18 hours. After the reaction mixture was filtered, water was added to the filtrate, followed by extraction with dichloromethane. The dichloromethane layer was washed with 1 N hydrochloric acid, a saturated aqueous solution of sodium hydrogencarbonate and a saturated aqueous solution of sodium chloride, dried over anhydrous sodium sulfate, and then concentrated under reduced pressure. The resulting crude product was dissolved in a small quantity of ethyl acetate and the solution was stirred at 0°C for one hour. After the reaction mixture was filtered, the filtrate was concentrated under reduced pressure. The resulting crude product was purified by silica gel column chromatography (50 ml of silica gel, chloroform : methanol = 10 : 1) to give 84 mg (yield 64%) of Compound 5.

The physicochemical properties of Compound 5 are as follows.

| | |
|---|---|
| $^1$H-NMR (400MHz, $CD_3OD$) δ(ppm); | 8.49(1H, br s), 8.25 (1H, s), 7.04(1H, br s), 6.96(1H, s), 5.47(1H, br), 4.68(1H, dd, J = 11.0, 9.6Hz), 4.51(1H, dd, J = 11.0, 4.0Hz), 4.19(1H, m), 4.04(3H, s), 3.94 (1H, dd, J = 10.1, 3.2Hz), 3.88(3H, s), 3.87(3H, s), 3.83(1H, dd, J = 10.1, 7.1Hz), 3.68(3H, s), 3.62 (4H, br), 2.66(4H, m), 2.59(4H, m), 2.37(3H, s), 1.57(3H, s) |
| IR (KBr) ν ($cm^{-1}$); | 2936, 1738, 1617, 1539, 1458, 1411, 1378, 1305, 1221, 1117, 1023, 976 |
| SIMS (m/z); | 772, 770$(M+1)^+$, 690, 234 |

Example 6 Synthesis of Compound 6

In 10 ml of dichloromethane was dissolved 100 mg (0.17 mmol) of DC-89B2, and 5 ml of a solution of 53 mg (0.26 mmol) of DCC in dichloromethane was added to the solution at 0°C. The mixture was stirred at 0°C for 0.5 hour. Then, 55 mg (0.26 mmol) of 4-(4-methyl-1-piperazinyl-carbonyl)butyric acid was added to the reaction mixture, and the mixture was stirred at 0°C to room temperature for 18 hours. After the reaction mixture was filtered, water was added to the filtrate, followed by extraction with dichloromethane. The dichloromethane layer was washed with 1 N hydrochloric acid, a saturated aqueous solution of sodium hydrogencarbonate and a saturated aqueous solution of sodium chloride, dried over anhydrous sodium sulfate, and then concentrated under reduced pressure. The resulting crude product was dissolved in a small quantity of ethyl acetate and the solution was stirred at 0°C for one hour. After the reaction mixture was filtered, the filtrate was concentrated under reduced pressure. The resulting crude product was purified by silica gel column chromatography (40 ml of silica gel, chloroform : methanol = 20 : 1) to give 122 mg (yield 91%) of Compound 6.

The physicochemical properties of Compound 6 are as follows.

| | |
|---|---|
| $^1$H-NMR (400MHz, $CDCl_3$) δ (ppm); | 9.33(1H, br s), 8.42 (1H, s), 6.95(1H, d, J = 2.3Hz), 6.87(1H, s), 6.27 (1H, br), 4.62(1H, br d, J = 9.1Hz), 4.57(1H, dd, J = 10.8, 4.6Hz), 4.23(1H, m), 4.08(3H, s),3.94(3H, s), 3.91(3H, s), 3.75(3H, |

| | |
|---|---|
| | s), 3.71(6H, m), 2.75 (2H, m), 2.54(4H, m), 2.42(3H, s), 2.41(2H, m), 2.13(2H, m), 1.67(3H, s) |
| IR (KBr) ν ($cm^{-1}$); | 2938, 1734, 1614, 1526, 1460, 1440, 1369, 1304, 1264, 1115, 995 |
| SIMS (m/z); | 786, 784$(M+1)^+$, 234 |

Example 7 Synthesis of Compound 7

In 8 ml of dichloromethane was dissolved 50 mg (0.085 mmol) of DC-89B2, and 0.024 ml (0.17 mmol) of triethylamine and 43 mg (0.21 mmol) of p-nitrophenyl chloroformate were added to the solution at 0°C. The mixture was stirred at 0°C for one hour. Then, 0.021 ml (0.25 mmol) of pyrrolidine was added to the reaction mixture, followed by stirring at 0°C for 2 hours. After 1 N hydrochloric acid was added to the reaction mixture, the mixture was extracted with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (30 ml of silica gel, n-hexane : ethyl acetate = 1 : 1) to give 51 mg (yield 87%) of Compound 7.

The physicochemical properties of Compound 7 are as follows.

| | |
|---|---|
| $^1$H-NMR (400MHz, $CDCl_3$) δ (ppm); | 9.32(1H, br s), 8.45 (1H, s), 6.95(1H, d, J=2.4Hz), 6.87(1H, s), 5.59 (1H, br s), 4.64(1H, br d, J=9.4Hz), 4.58(1H, dd, J=10.4, 4.5Hz), 4.25(1H, m), 4.08(3H, s), 4.05 (1H, dd, J=10.1, 3.4Hz), 3.94(3H, s), 3.91(3H, s), 3.77(3H, s), 3.63(2H, t, J=6.7Hz), 3.60-(1H, dd, J=10.1, 9.9Hz), 3.52(2H, t, J=6.7Hz), 1.98(4H, m), 1.68-(3H, s) |
| IR (KBr) ν ($cm^{-1}$); | 2941, 1705, 1616, 1539, 1521, 1492, 1386, 1306, 1166, 1109, 1051 |
| SIMS (m/z); | 687, 685$(M+1)^+$, 453, 451, 234 |

Example 8 Synthesis of Compound 8

In 8 ml of dichloromethane was dissolved 50 mg (0.085 mmol) of DC-89B2, and 0.024 ml (0.17 mmol) of triethylamine and 43 mg (0.21 mmol) of p-nitrophenyl chloroformate were added to the solution at 0°C. The mixture was stirred at 0°C for one hour. Then, 0.025 ml (0.25 mmol) of piperidine was added to the reaction mixture, followed by stirring at 0°C for 2 hours. After 1 N hydrochloric acid was added to the reaction mixture, the mixture was extracted with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (30 ml of silica gel, n-hexane : ethyl acetate = 1 : 1) to give 45 mg (yield 75%) of Compound 8.

The physicochemical properties of Compound 8 are as follows.

| | |
|---|---|
| $^1$H-NMR (400MHz, $CDCl_3$) δ (ppm); | 9.33(1H, br s), 8.44 (1H, s), 6.96(1H, d, J=2.3Hz), 6.87(1H, s), 5.52 (1H, br), 4.62(1H, br d, J=9.2Hz), 4.58(1H, dd, J=10.8, 4.5Hz), 4.26(1H, m), 4.08(3H, s), 4.04 (1H, dd, J=10.1, 3.4Hz), 3.94(3H, s), 3.91(3H, s), 3.78(3H, s), 3.66(2H, br), 3.60(1H, dd, J=10.0, 9.0Hz), 3.54(2H, br), 2.27(6H, m), 1.68(3H, s) |
| IR (KBr) ν ($cm^{-1}$); | 2940, 1702, 1621, 1523, 1494, 1429, 1387, 1306, 1228, 1141, 1049 |
| SIMS (m/z); | 701, 699$(M+1)^+$, 234 |

Example 9 Synthesis of Compound 9

In 8 ml of dichloromethane was dissolved 50 mg (0.085 mmol) of DC-89B2, and 0.024 ml (0.17 mmol) of triethylamine and 43 mg (0.21 mmol) of p-nitrophenyl chloroformate were added to the solution at 0°C. The mixture was stirred at 0°C for one hour. Then, 0.022 ml (0.25 mmol) of morpholine was added to the reaction mixture, followed by stirring at 0°C for 2 hours. After 1 N hydrochloric acid was added to the reaction mixture, the mixture was extracted with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (50 ml of silica gel, n-hexane :

ethyl acetate = 1 : 2) to give 50 mg (yield 84%) of Compound 9.

The physicochemical properties of Compound 9 are as follows.

| | |
|---|---|
| $^1$H-NMR (400MHz, $CDCl_3$) δ(ppm); | 9.31(1H, br s), 8.46 (1H, s), 6.96(1H, d, J=2.3Hz), 6.87(1H, s), 5.47 (1H, br s), 4.63(1H, br d, J=9.3Hz), 4.59(1H, dd, J=10.7, 4.4Hz), 4.26(1H, m), 4.08(3H, s), 4.04 (1H, dd, J=10.1, 3.3Hz), 3.94(3H, s), 3.91(3H, s), 3.78(3H, s), 3.77(4H, m), 3.73(2H, br), 3.62(1H, dd, J=10.0, 4.4Hz), 3.61(2H, br), 1.69(3H, s) |
| IR (KBr) ν ($cm^{-1}$); | 2940, 1714, 1616, 1520, 1494, 1429, 1386, 1306, 1231, 1115, 1051 |
| SIMS (m/z); | 703, 701$(M+1)^+$, 469, 467, 234 |

Example 10 Synthesis of Compound 10

In 5 ml of dichloromethane was dissolved 30 mg (0.051 mmol) of DC-89B2, and 0.014 ml (0.1 mmol) of triethylamine and 26 mg (0.13 mmol) of p-nitrophenyl chloroformate were added to the solution at 0°C. The mixture was stirred at 0°C for one hour. Then, 0.017 ml (0.15 mmol) of N-methylpiperazine was added to the reaction mixture, followed by stirring at 0°C for 2 hours. After 1 N hydrochloric acid was added to the reaction mixture, the mixture was extracted with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (30 ml of silica gel, chloroform : methanol = 50 : 1) to give 35 mg (yield 96%) of Compound 10.

The physicochemical properties of Compound 10 are as follows.

| | |
|---|---|
| $^1$H-NMR (400MHz, $CDCl_3$) δ (ppm); | 9.32(1H, br s), 8.45 (1H, s), 6.94(1H, d, J=2.3Hz), 6.87(1H, s), 5.48 (1H, br s), 4.63(1H, br d, J=9.3Hz), 4.59(1H, dd, J=10.8, 4.5Hz), 4.25(1H, m), 4.08(3H, s), 4.04 (1H, dd, J=10.1, 3.3Hz), 3.94 (3H, s), 3.92(3H, s), 3.78(3H, s), 3.76(2H, br), 3.64(2H, br), 3.61 (1H, dd, J=10.0, 8.9Hz), 2.52(4H, br), 2.37(3H, s), 1.69(3H, s) |
| IR (KBr) ν ($cm^{-1}$); | 2940, 1710, 1621, 1521, 1493, 1430, 1385, 1289, 1231, 1049, 1002 |
| SIMS (m/z); | 716, 714$(M+1)^+$, 482, 480, 234 |

Example 11 Synthesis of Compound 11

In 3 ml of dichloromethane was dissolved 30 mg (0.051 mmol) of DC-89B2, and 1 ml of a solution of 16 mg (0.078 mmol) of DCC in dichloromethane was added to the solution at 0°C, followed by stirring at 0°C for 0.5 hour. After 16 mg (0.076 mmol) of 4-(4-morpholino)butyric acid hydrochloride was added to the reaction mixture, the mixture was stirred at 0°C to room temperature for 18 hours. The reaction mixture was filtered and the filtrate was concentrated under reduced pressure. The resulting crude product was dissolved in a small quantity of ethyl acetate and the solution was stirred at 0°C for one hour. The reaction mixture was filtered and the filtrate was concentrated under reduced pressure. The resulting crude product was purified by silica gel column chromatography (50 ml of silica gel, chloroform : methanol = 5 : 1) to give 27.9 mg (yield 74%) of Compound 11.

The physicochemical properties of Compound 11 are as follows.

| | |
|---|---|
| $^1$H-NMR (400MHz, DMSO-$d_6$) δ (ppm); | 11.32(1H, br s), 7.88(1H, s), 7.00(1H, d, J=2.0Hz), 6.98(1H, s), 4.68(1H, dd, J=10.4, 10.4Hz), 4.34(1H, dd, J= 10.4, 5.7Hz), 4.19(1H, m), 4.01(1H, br), 3.94(3H, s), 3.81(3H, s), 3.80(3H, s), 3.79(5H, m), 3.63 (3H, s), 3.09(1H, br), 2.95(6H, br), 2.79(2H, br), 1.78(2H, br), 1.49(3H, s) |
| IR (KBr) ν ($cm^{-1}$); | 2941, 1745, 1652, 1620, 1529, 1490, 1386, 1306, 1110 |
| SIMS (m/z); | 745, 743$(M+1)^+$, 590, 588, 234 |

Example 12 Synthesis of Compound 12

In 2 ml of dichloromethane was dissolved 20 mg (0.034 mmol) of DC-89B2, and 1 ml of a solution of 11 mg (0.053 mmol) of DCC in dichloromethane was added to the solution at 0°C, followed by stirring at 0°C for 0.5 hour. After 11.5 mg (0.051 mmol) of 5-(4-morpholino)valeric acid hydrochloride was added to the

reaction mixture, the mixture was stirred at 0 ° C to room temperature for 18 hours. The reaction mixture was filtered and the filtrate was concentrated under reduced pressure. The resulting crude product was dissolved in a small quantity of ethyl acetate and the solution was stirred at 0 ° C for one hour. The reaction mixture was filtered and the filtrate was concentrated under reduced pressure. The resulting crude product was purified by silica gel column chromatography (30 ml of silica gel, chloroform : methanol = 5 : 1) to give 9 mg (yield 35%) of Compound 12.

The physicochemical properties of Compound 12 are as follows.

| | |
|---|---|
| $^1$H-NMR (400MHz, DMSO-$d_6$) δ (ppm); | 11.35(1H, br s), 7.85 (1H, s), 7.01(1H, d, J = 1.9Hz), 6.97(1H, s), 4.69 (1H, dd, J = 10.3, 10.3Hz), 4.35(1H, dd, J = 10.3, 4.3Hz), 4.20(1H, m), 4.03(1H, br), 3.93(3H, s), 3.82(3H, s), 3.80(3H, s), 3.65(5H, m), 3.64(3H, s), 3.30(1H, br), 2.73(6H, br), 2.34(2H, br), 1.71(4H, br), 1.50(3H, s) |
| IR (KBr) ν ($cm^{-1}$); | 2930, 1743, 1652, 1612, 1520, 1489, 1387, 1306, 1112 |
| SIMS (m/z); | 759, 757$(M+1)^+$, 677, 590, 588, 234 |

Example 13 Synthesis of Compound 13

In 4 ml of dichloromethane was dissolved 20 mg (0.034 mmol) of DC-89B2, and 0.012 ml (0.086 mmol) of triethylamine and 0.009 ml (0.083 mmol) of phenyl isocyanate were added to the solution at 0 ° C. The mixture was stirred at 0 ° C for one hour. After 1 N hydrochloric acid was added to the reaction mixture, the mixture was extracted with chloroform. The chloroform layer was washed with a saturated aqueous solution of sodium hydrogencarbonate and a saturated aqueous solution of sodium chloride, dried over anhydrous sodium sulfate, and then concentrated under reduced pressure. The resulting crude product was purified by silica gel column chromatography (30 ml of silica gel, n-hexane : ethyl acetate = 1 : 1) to give 19.7 mg (yield 82%) of Compound 13.

The physicochemical properties of Compound 13 are as follows.

| | |
|---|---|
| $^1$H-NMR (400MHz, $CDCl_3$) δ (ppm); | 9.37(1H, br s), 8.56 (1H, s), 7.47(2H, d, J = 7.0Hz), 7.36(2H, m), 7.15 (2H, m), 6.95(1H, d, J = 2.3Hz), 6.87(1H, s), 5.44 (1H, s), 4.62(1H, br d, J = 9.4Hz), 4.58(1H, dd, J = 10.7, 4.5Hz), 4.24(1H, m), 4.07(3H, s), 4.04(1H, dd, J = 10.0, 3.5Hz), 3.94(3H, s), 3.91(3H, s), 3.78(3H, s), 3.61(1H, dd, J = 10.0, 9.0Hz), 1.69(3H, s) |
| IR (KBr) ν ($cm^{-1}$); | 2941, 1734, 1602, 1524, 1442, 1388, 1308, 1193, 1040 |
| SIMS (m/z); | 709, 707$(M+1)^+$, 234 |

Example 14 Synthesis of Compound 14

In 1.5 ml of ethanol was dissolved 29 mg (0.041 mmol) of Compound 10, and 0.01 ml of 5.8 N hydrogen chloride-ethanol was added to the solution, followed by stirring at 0 ° C for one hour. After 30 ml of diethyl ether was added to the reaction mixture, the mixture was stirred at 0 ° C for one hour. The formed crystals were taken by filtration under reduced pressure to give 16.3 mg (yield 53%) of Compound 14.

The physicochemical properties of Compound 14 are as follows.

| | |
|---|---|
| $^1$H-NMR (400MHz, DMSO-$d_6$) δ (ppm); | 11.29(1H, br s), 10.40(1H, br), 8.30(1H, br s), 7.83(1H, s), 7.00 (1H, d, J = 2.2Hz), 6.96(1H, s), 4.68(1H, dd, J = 10.4, 10.4Hz), 4.38(2H, br), 4.34(1H, dd, J = 10.4, 4.4Hz), 4.20(1H, m), 4.18(2H, br), 3.97(1H, dd, J = 10.0, 3.1Hz), 3.93(3H, s), 3.90(1H, dd, J = 10.0, 6.7Hz), 3.81(3H, s), 3.79(3H, s), 3.48(2H, br), 3.20(2H, br), 2.83(3H, s), 1.50(3H, s) |
| IR (KBr) ν ($cm^{-1}$); | 2930, 1718, 1617, 1522, 1492, 1457, 1430, 1387, 1308, 1234, 1170, 1109 |

Example 15 Synthesis of Compound 15

In 5 ml of dichloromethane was dissolved 50 mg (0.092 mmol) of DC-89A2, and 0.026 ml (0.19 mmol) of triethylamine and 37 mg (0.18 mmol) of p-nitrophenyl chloroformate were added to the solution at 0 ° C. The mixture was stirred at 0 ° C for one hour. Then, 0.031 ml (0.28 mmol) of N-methylpiperazine was added to the reaction mixture, followed by stirring at 0 ° C for 2 hours. After 1 N hydrochloric acid was added to the reaction mixture, the mixture was extracted with chloroform. The chloroform layer was washed with a saturated aqueous solution of sodium hydrogen-carbonate and a saturated aqueous solution of sodium

chloride, dried over anhydrous sodium sulfate, and then concentrated under reduced pressure. The resulting crude product was purified by silica gel column chromatography (30 ml of silica gel, chloroform : methanol = 50 : 1) to give 60 mg (yield 98%) of Compound 15.

The physicochemical properties of Compound 15 are as follows.

| | |
|---|---|
| $^1$H-NMR (400MHz, $CDCl_3$) $\delta$ (ppm); | 9.32(1H, br s), 8.45 (1H, s), 6.96(1H, d, J=2.4Hz), 6.87(1H, s), 5.47 (1H, br s), 4.63(2H, m), 4.22(1H, m), 4.16(1H, dd, J=10.7, 3.5Hz), 4.08(3H, s), 3.94(3H, s), 3.91(3H, s), 3.81(2H, br), 3.78(3H, s), 3.74(1H, dd, J=10.7, 8.4Hz), 3.70(2H, br), 2.58(4H, br), 2.43-(3H, br s), 1.69(3H, s) |
| IR (KBr) $\nu$ ($cm^{-1}$); | 1711, 1618, 1495, 1431, 1308, 1293, 1230, 1148 |
| SIMS (m/z); | $669(M)^+$, 234 |

## Claims

**1.** A DC-89 derivative represented by the formula:

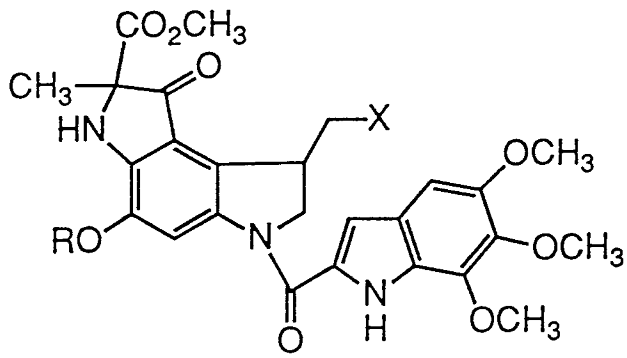

wherein X represents chlorine or bromine; and R represents

$-SO_2R^1$ in which $R^1$ represents a straight-chain or branched alkyl group having 1 to 6 carbon atoms or phenyl group, or

$-CO(CH_2)_nR^2$ in which n represents an integer of 0 to 5, and $R^2$ represents

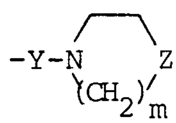

in which m represents an integer of 1 to 4;

Y represents a single bond or CO; and Z represents $CH_2$, O or N-$R^3$ (in which $R^3$ represents hydrogen or a straight-chain or branched alkyl group having 1 to 6 carbon atoms) or

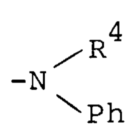

in which $R^4$ represents hydrogen or a straight-chain or branched alkyl group having 1 to 6 carbon atoms, and Ph represents phenyl group,

or a pharmaceutically acceptable salt thereof.

**2.** A compound according to Claim 1, wherein R represents $CO(CH_2)_nR^2$.

**3.** A compound according to Claim 2, wherein n represents 0.

4. A compound according to Claim 3, wherein $R^2$ represents

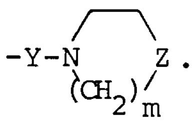

5. A compound according to Claim 4, wherein Y represents a single bond.

6. A compound according to Claim 5, wherein m represents 2.

7. A compound according to Claim 6, wherein Z represent N-$R^3$.

8. A compound according to Claim 7, wherein $R^3$ represents a straight-chain or branched alkyl group having 1 to 6 carbon atoms.

9. A compound according to Claim 7, wherein $R^3$ represents methyl.

10. A compound according to Claim 3, wherein $R^2$ represents

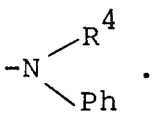

11. A compound according to Claim 10, wherein $R^4$ represents hydrogen.

12. A pharmaceutical composition comprising a pharmaceutically acceptable carrier and as an active ingredient, an effective amount of a compound as defined by any one of Claims 1-11.

13. A compound according to any one of Claims 1-11 for the use as a medicament.

14. Use of a compound according to anyone of Claims 1-11 or of the composition according to Claim 12 for the manufacture of a medicament having anti-tumor activity.

European Patent Office

# EUROPEAN SEARCH REPORT

Application Number

EP 92 10 1913

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 339 681 (MEIJI SEIKA KAISHA)<br>* claim 1 * | 1,12 | C07D487/04<br>A61K31/40<br>//(C07D487/04, 209:00,209:00) |
| D,A | EP-A-0 365 041 (KYOWA HAKKO KOGYO)<br>* claims 1,17 * | 1,12 | |
| P,A ; D | EP-A-0 461 603 (KYOWA HAKKO KOGYO)<br>* claims 1,8 * | 1,12 | |

TECHNICAL FIELDS SEARCHED (Int. Cl.5 )

C07D
A61K

The present search report has been drawn up for all claims

| Place of search | Date of completion of the search | Examiner |
|---|---|---|
| THE HAGUE | 19 MAY 1992 | ALFARO FAUS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document